Europäisches Patentamt

European Patent Office

Office européen des brevets

(19)

(11) Numéro de publication : **0 001 192**
**B1**

(12) **FASCICULE DE BREVET EUROPÉEN**

(45) Date de publication du fascicule du brevet :
23.12.81

(21) Numéro de dépôt : 78400084.6

(22) Date de dépôt : 30.08.78

(51) Int. Cl.³ : **C 08 L   5/00**, C 08 L   5/14// A61L9/01, A23L1/04, C06B47/14

(54) **Compositions gélifiantes à base de galactomannanes et de xanthane désacétylé et procédé d'obtention.**

(30) Priorité : 12.09.77 FR 7727467

(43) Date de publication de la demande :
21.03.79 (Bulletin 79/06)

(45) Mention de la délivrance du brevet :
23.12.81 Bulletin 81/51

(84) Etats contractants désignés :
CH DE GB NL

(56) Documents cités :
US - A - 3 384 498
US - A - 3 677 961

(73) Titulaire : **C E C A S.A.**
**11, Avenue Morane Saulnier**
**F-78140 - Velizy Villacoublay (FR)**

(72) Inventeur : **Brigand, Gérard**
**Cité Auby**
**F-50500 Carentan (FR)**
Inventeur : **Kragen, Horst**
**Cité Auby**
**F-50500-Carentan (FR)**

(74) Mandataire : **Casalonga, Alain et al**
**Bureau D.A. Casalonga Office Josse & Petit Lilien-strasse 77**
**D-8000 München 80 (DE)**

Imprimerie Jouve, 17, rue du Louvre, 75001 Paris, France

# 0 001 192

### Compositions gélifiantes à base de galactomannane et xanthane désacétylé et procédé d'obtention

La présente invention concerne les compositions donnant des gels aqueux, à base de galactomannane et de xanthane désacétylé, ainsi que les gels obtenus avec ces compositions et leur application.

On connaît notamment comme agents épaississants les galactomannanes qui sont les composants des gommes extraites de végétaux telles que les gommes de guar, de caroube, tara, d'Espina corona.

On connaît aussi les agents gélifiants obtenus à partir de glucides tels que les sucres par l'action d'un micro-organisme ; tel est le cas du xanthane, polysaccharide obtenu par l'action de bactéries du genre Xanthomonas.

Il est apparu qu'en associant du xanthane et des galactomannanes, on pouvait obtenir des gels de qualité améliorée ; de tels gels ont été décrits avec la gomme de caroube dans les brevets US 3 557 016, 3 726 690, 3 519 434, 3 623 868, avec la gomme tara dans le brevet français 2 119 365. Ces effets de synergie ont été notamment signalés dans l'article de J. K. Rocks « Xanthan gums » publié dans la revue « Food Technology », Volume 25, Mai 1971, page 2 231, qui signale que de tels effets n'ont pu être obtenus avec la gomme de guar. Toutefois, l'association de xanthane, ayant subi un traitement thermique, et d'une gomme de guar a été décrite dans le brevet français 2 193 074.

Au cours d'essais on a observé, de façon inattendue, que la désacétylation préalable du xanthane permettait d'obtenir avec les diverses gommes de galactomannanes des gels beaucoup plus forts que ceux obtenus avec le xanthane et ceci quelles que soient les conditions de salinité et de pH des gels.

Le xanthane est un polysaccharide qui peut être considéré comme un ester acétylique d'un polymère contenant du mannose, du glucose et de l'acide glucuronique sous forme de sel de potassium. Les groupes acétyles représentent environ 4,7 % du polysaccharide ; un traitement alcalin permet d'éliminer ces groupes acétylés et d'obtenir un xanthane désacétylé partiellement ou totalement. Le procédé de désacétylation a notamment été décrit dans le brevet US 3 000 790 qui indique que le xanthane désacétylé permet d'obtenir des solutions visqueuses moins sensibles aux sels inorganiques que le xanthane naturel.

Il a été trouvé, et ceci est l'objet de l'invention, que l'association xanthane désacétylé avec un galactomannane donnait, par effet de synergie, des gels de force plus grande que l'association xanthane naturel-galactomannane ; en particulier, alors que l'association xanthane naturel-gomme de guar donne, par un effet de synergie, des solutions de forte viscosité pouvant aller jusqu'à la formation d'un gel très faible, on a observé qu'avec le xanthane désacétylé on obtenait des gels forts.

Cette dernière interaction représente un avantage important de l'invention.

L'invention sera mieux comprise à l'aide des exemples suivants et des figures annexées 1 à 12 qui représentent des courbes de cohésion et de rigidité des sels en fonction du rapport de mélange et de l'influence de certains agents.

Dans ces exemples, le xanthane naturel est appelé xanthane acétylé pour bien marquer la différence avec le xanthane désacétylé, le terme xanthane pouvant désigner l'un ou l'autre des deux produits.

La viscosité (η) des xanthanes, des galactomannanes ou de leur association est mesurée à 20 °C au viscosimètre Brookfield à 20 tr/mn sur une solution à 1 % et est exprimée en milli-Pascal secondes (mPa.s).

Les gels de l'association xanthane-galactomannane sont obtenus par dispersion à froid à la dose de 1 %, suivie d'un chauffage entre 75 et 95 °C et d'un refroidissement.

Ces gels ont été caractérisés par :

— leur rigidité : force nécessaire pour faire pénétrer un piston d'une course de 8 mm à l'intérieur du gel, mesurée à l'appareil Bloom Gelometer ; elle est exprimée en grammes.

— leur cohésion : résistance à l'écrasement mesurée à l'appareil dit de Kobé, en ajoutant des poids de 200 g toutes les 15 secondes ; elle est exprimée en kilogrammes.

### Exemple 1 : Augmentation de la synergie entre xanthane et gomme de caroube

On a utilisé un xanthane acétylé et un xanthane désacétylé de viscosité 1 000 mPa.s environ et une gomme de caroube de viscosité 1 300 mPa.s environ.

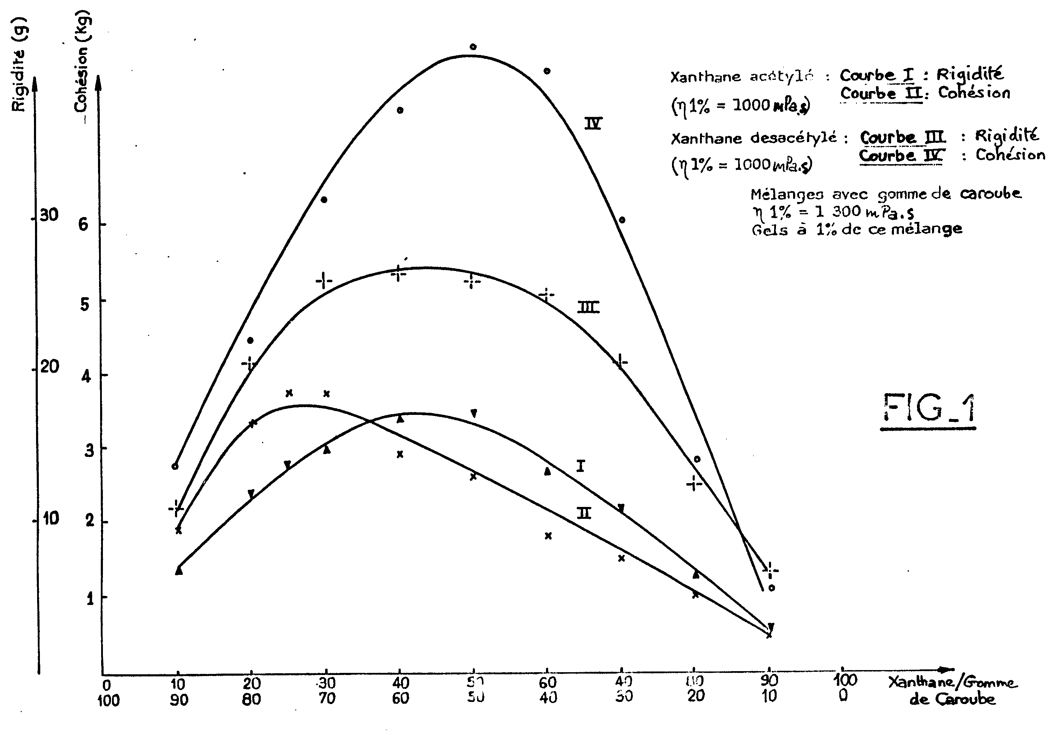

La comparaison entre le xanthane acétylé et le xanthane désacétylé, pour différents rapports xanthane/gomme de caroube est montrée dans le tableau I et sur la figure 1.

Voir Tableau page 3

## 0 001 192

### TABLEAU I

| Rapport xanthane/gomme de caroube | Rigidité (g) | | Cohésion (kg) | |
|---|---|---|---|---|
| | Xanthane acétylé | Xanthane désacétylé | Xanthane acétylé | Xanthane désacétylé |
| 10/90 | 14 | 22 | 1,9 | 2,8 |
| 20/80 | 23 | 41 | 3,3 | 4,4 |
| 30/70 | 30 | 52 | 3,7 | 6,3 |
| 40/60 | 34 | 53 | 2,9 | 7,5 |
| 50/50 | 34 | 52 | 2,6 | 8,3 |
| 50/40 | 27 | 50 | 1,8 | 8 |
| 70/30 | 21 | 41 | 1,5 | 6 |
| 80/20 | 13 | 25 | 1 | 2,8 |
| 90/10 | 5 | 13 | 0,5 | 1,1 |

Exemple 2 : Influence du degré de polymérisation de la gomme de caroube

L'augmentation de synergie se manifeste quel que soit le degré de polymérisation de la gomme de caroube.

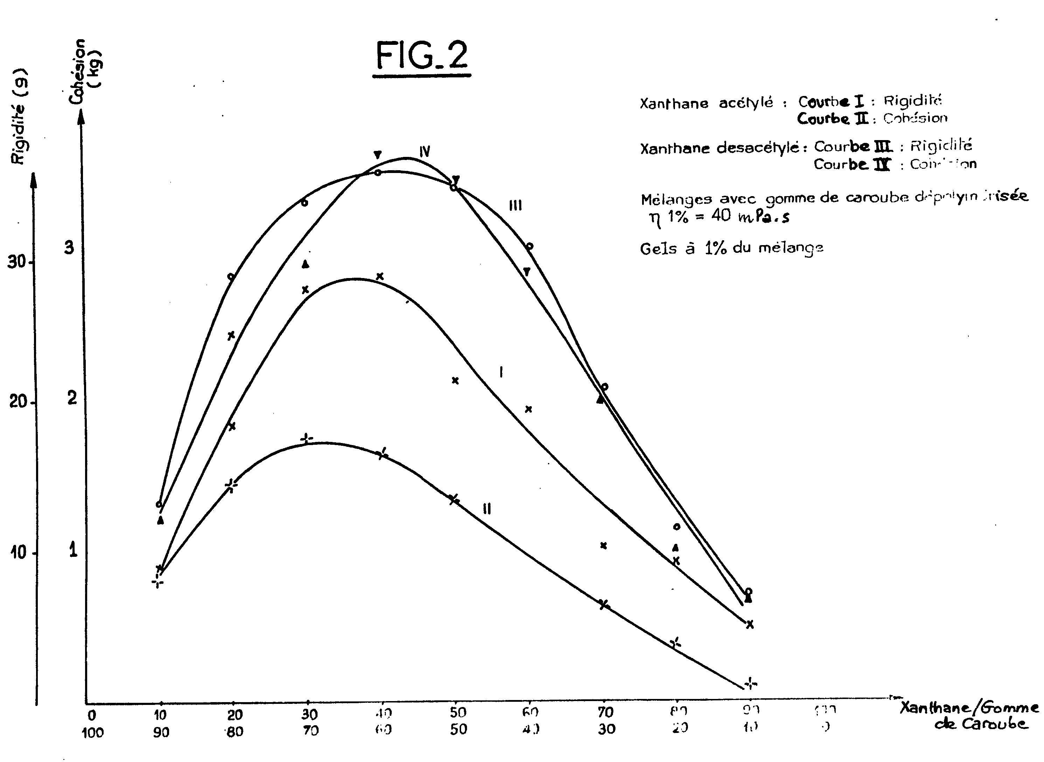

L'essai fait avec une gomme de caroube de viscosité 40 cP donne les résultats du tableau II et de la figure 2.

### TABLEAU II

| Rapport xanthane/gomme de caroube | Rigidité (g) | | Cohésion (kg) | |
|---|---|---|---|---|
| | Xanthane acétylé | Xanthane désacétylé | Xanthane acétylé | Xanthane désacétylé |
| 10/90 | 8 | 13 | 0,8 | 1,2 |
| 20/80 | 18 | 28 | 1,4 | 2,4 |
| 30/70 | 27 | 33 | 1,7 | 2,9 |
| 40/60 | 28 | 35 | 1,6 | 3,6 |
| 50/50 | 21 | 34 | 1,3 | 3,4 |
| 60/40 | 19 | 30 | 0,9 | 2,8 |
| 70/30 | 10 | 20 | 0,6 | 2,1 |
| 80/20 | 9 | 11 | 0,35 | 1 |
| 90/10 | 7 | 7 | 0,1 | 0,7 |

Exemple 3 : Amélioration de la synergie entre xanthane et gomme de guar

On a utilisé un xanthane acétylé et un xanthane désacétylé de viscosité 1 300 mPa.s et une gomme de guar de viscosité 3 500 mPa.s.

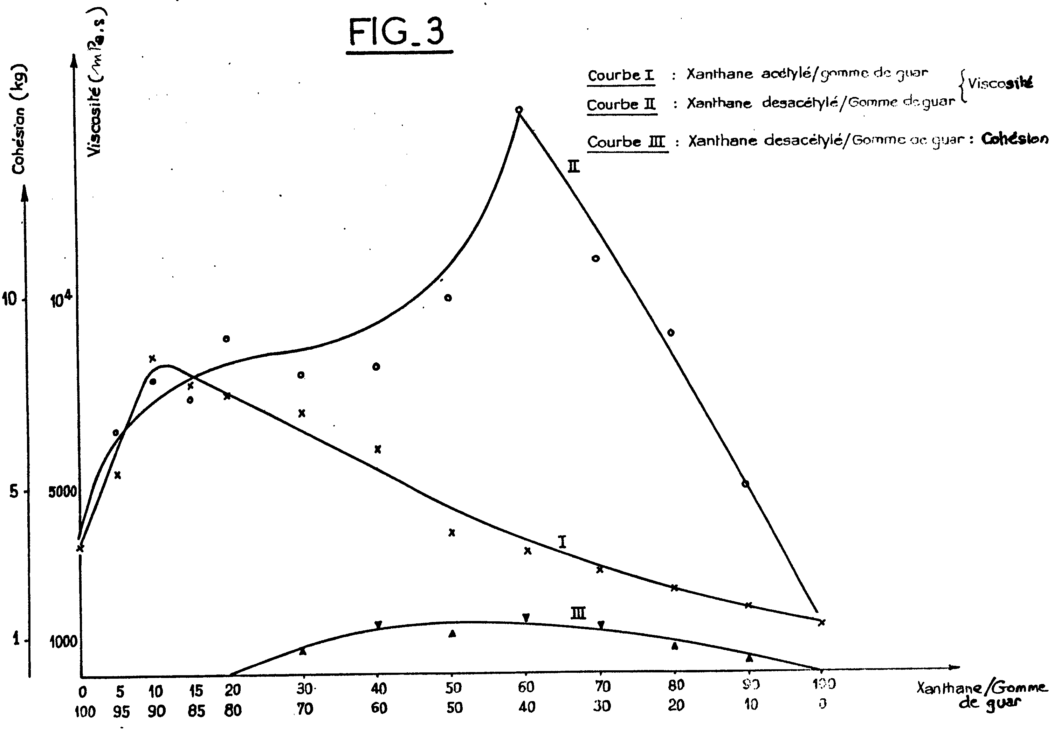

La comparaison entre le xanthane acétylé et le xanthane désacétylé, pour différents rapports xanthane/gomme de guar est montrée dans le tableau III et sur la figure 3.

### TABLEAU III

| Rapport xanthane/gomme de guar | Viscosité de la solution ou du gel broyé | | Cohésion du gel (kg) | |
|---|---|---|---|---|
| | Xanthane acétylé | Xanthane désacétylé | Xanthane acétylé | Xanthane désacétylé |
| 5/95 | 5 400 | 6 500 | liquide | liquide |
| 10/90 | 8 500 | 7 900 | liquide | liquide |
| 15/85 | 7 800 | 7 400 | gel non mesurable | liquide |
| 20/80 | 7 500 | 9 000 | liquide | liquide |
| 30/70 | 7 000 | 8 000 | liquide | 0,8 |
| 40/60 | 6 000 | 8 200 | liquide | 1,3 |
| 50/50 | 3 800 | 10 000 | liquide | 1,2 |

3

# 0 001 192

TABLEAU III (suite)

| Rapport xanthane/gomme de guar | Viscosité de la solution ou du gel broyé | | Cohésion du gel (kg) | |
|---|---|---|---|---|
| | Xanthane acétylé | Xanthane désacétylé | Xanthane acétylé | Xanthane désacétylé |
| 60/40 | 3 300 | 15 000 | liquide | 1,4 |
| 70/30 | 2 800 | 11 000 | liquide | 1,2 |
| 80/20 | 2 300 | 9 000 | liquide | 0,8 |
| 90/10 | 1 800 | 5 000 | liquide | 0,4 |

Exemple 4 : Amélioration de la synergie entre xanthane et gomme tara

On a utilisé un xanthane acétylé et un xanthane désacétylé de viscosité 1 000 mPa.s et une gomme tara de viscosité 600 mPa.s.

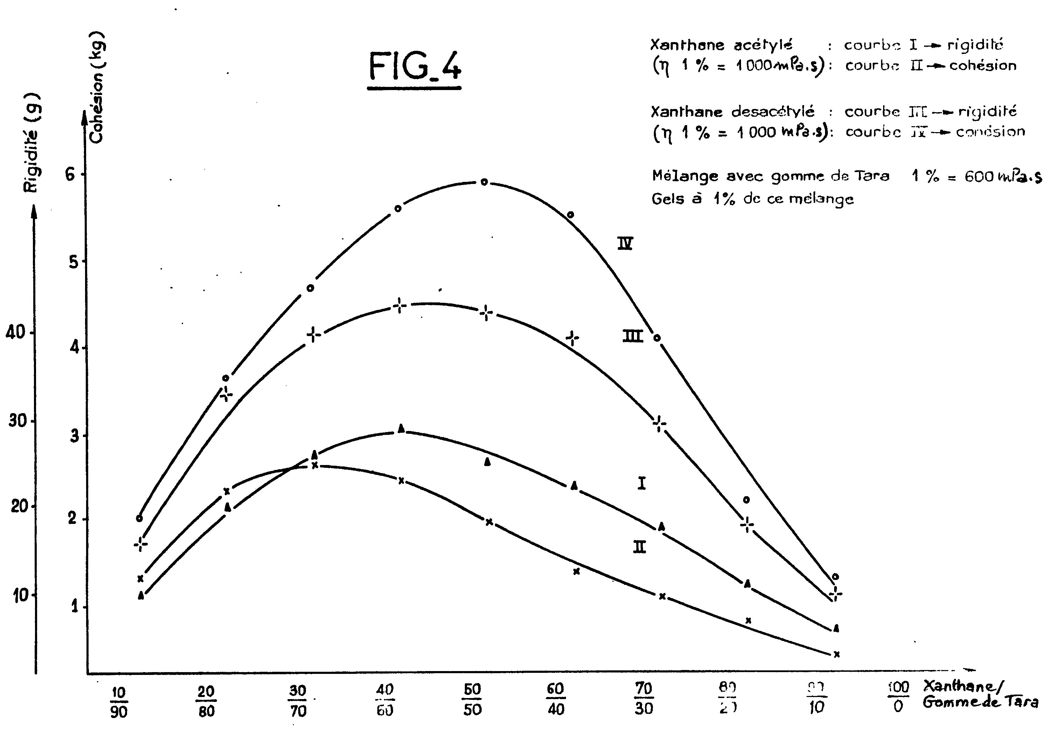

La comparaison entre le xanthane acétylé et le xanthane désacétylé, pour différents rapports xanthane/gomme tara est montrée dans le tableau IV et sur la figure 4.

TABLEAU IV

| Rapport xanthane/gomme tara | Rigidité (g) | | Cohésion (kg) | |
|---|---|---|---|---|
| | Xanthane acétylé | Xanthane désacétylé | Xanthane acétylé | Xanthane désacétylé |
| 10/90 | 11 | 17 | 1,3 | 2 |
| 20/80 | 21 | 34 | 2,3 | 3,6 |
| 30/70 | 27 | 41 | 2,6 | 4,6 |
| 40/60 | 30 | 44 | 2,4 | 5,5 |
| 50/50 | 26 | 43 | 1,9 | 5,8 |
| 60/40 | 23 | 40 | 1,3 | 5,4 |
| 70/30 | 18 | 30 | 1 | 4 |
| 80/20 | 11 | 18 | 0,7 | 2,1 |
| 90/10 | 6 | 10 | 0,3 | 1,2 |

Exemple 5 : Amélioration de la synergie entre xanthane et gomme d'Espina corona

On a utilisé un xanthane acétylé et un xanthane désacétylé de viscosité 1 000 mPa.s et une gomme d'Espina corona de viscosité 800 mPa.s.

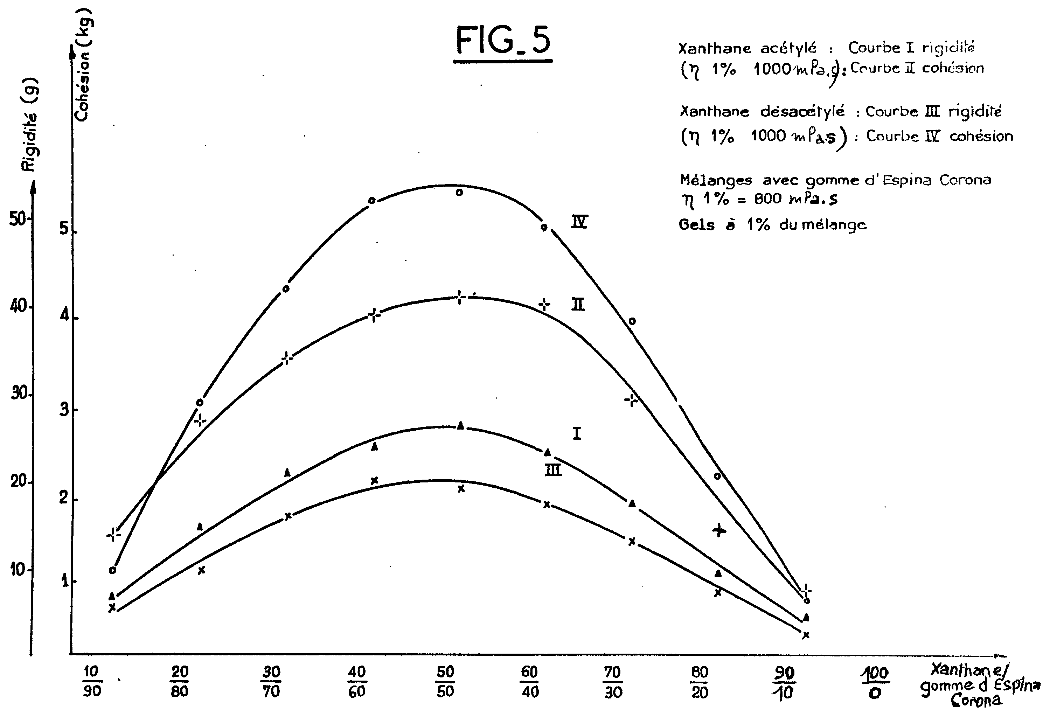

La comparaison entre le xanthane acétylé et le xanthane désacétylé, pour différents rapports xanthane/gomme d'Espina corona est montrée dans le tableau V et sur la figure 5.

TABLEAU V

| Rapport xanthane/gomme d'Espina corona | Rigidité (g) | | Cohésion (kg) | |
|---|---|---|---|---|
| | Xanthane acétylé | Xanthane désacétylé | Xanthane acétylé | Xanthane désacétylé |
| 10/90 | 8 | 15 | 0,7 | 1,1 |
| 20/80 | 16 | 28 | 1,1 | 3 |
| 30/70 | 22 | 35 | 1,7 | 4,3 |
| 40/60 | 25 | 40 | 2,1 | 5,3 |
| 50/50 | 27 | 42 | 2 | 5,4 |
| 60/40 | 24 | 41 | 1,8 | 5 |
| 70/30 | 18 | 30 | 1,4 | 3,9 |
| 80/20 | 10 | 15 | 0,8 | 2,1 |
| 90/10 | 5 | 8 | 0,3 | 0,7 |

4

### Exemple 6

De façon habituelle, l'utilisation de gels dans l'industrie ou dans l'alimentation demande un milieu contenant des sels, notamment du chlorure de sodium.

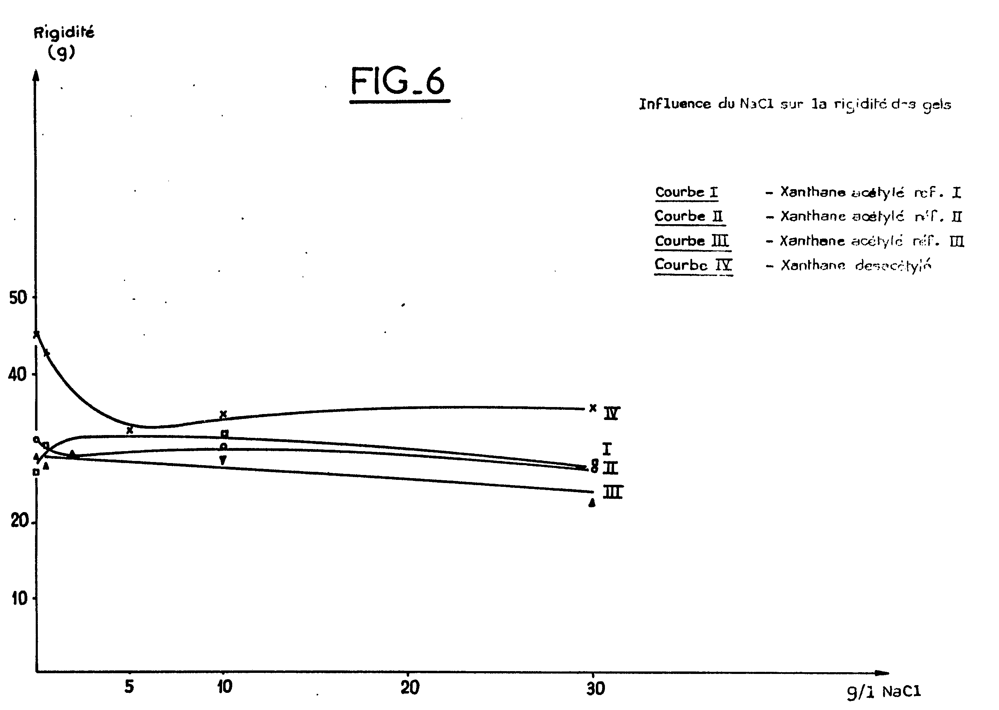

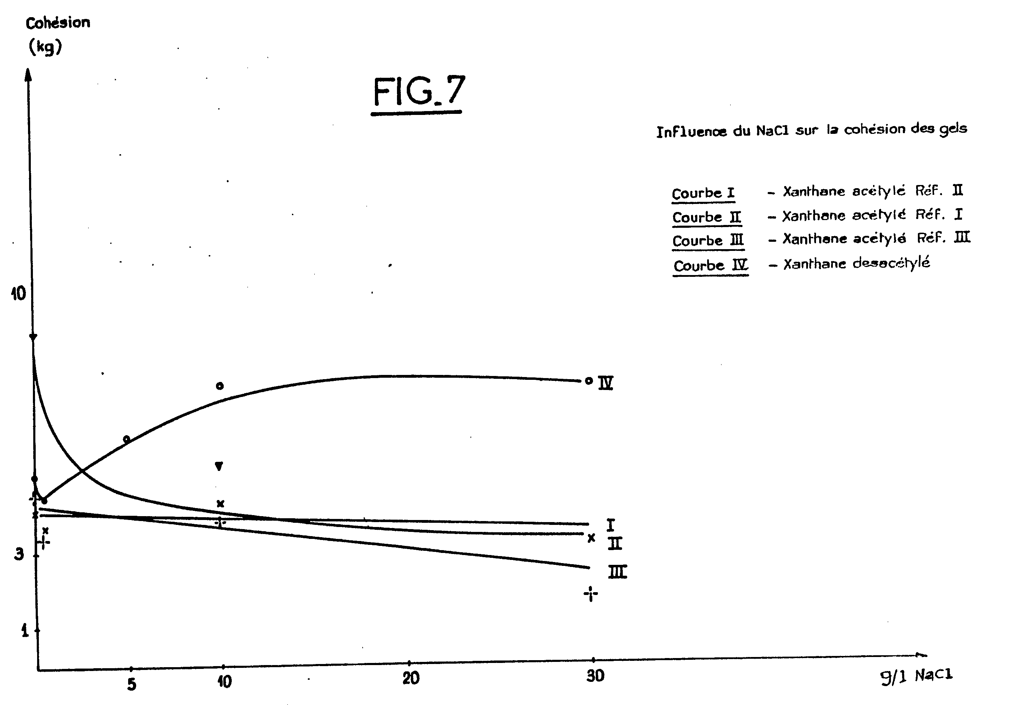

L'étude de l'influence des électrolytes tels que le chlorure de sodium a été faite avec un mélange 50/50 xanthane-gomme de caroube. Trois échantillons de xanthane acétylé ont été comparés au xanthane désacétylé, pour différentes doses de chlorure de sodium dans le gel. La figure 6 donne le résultat des mesures de rigidité et la figure 7 le résultat des mesures de cohésion.

Ces deux figures montrent la supériorité des gels à base de xanthane désacétylé. Cependant, du fait que les produits de départ n'ont pas forcément les mêmes caractéristiques (viscosité Brookfield), un exemple plus rigoureux consiste à partir de xanthane naturel désacétylé progressivement à la soude 0,015 N (on suit la désacétylation dans le temps).

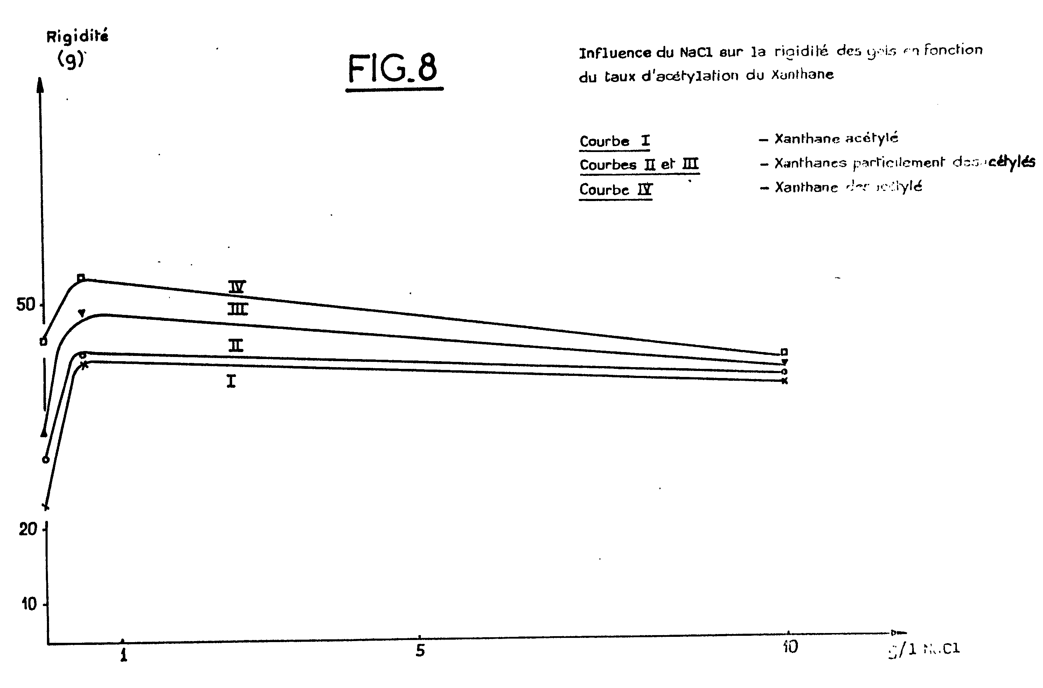

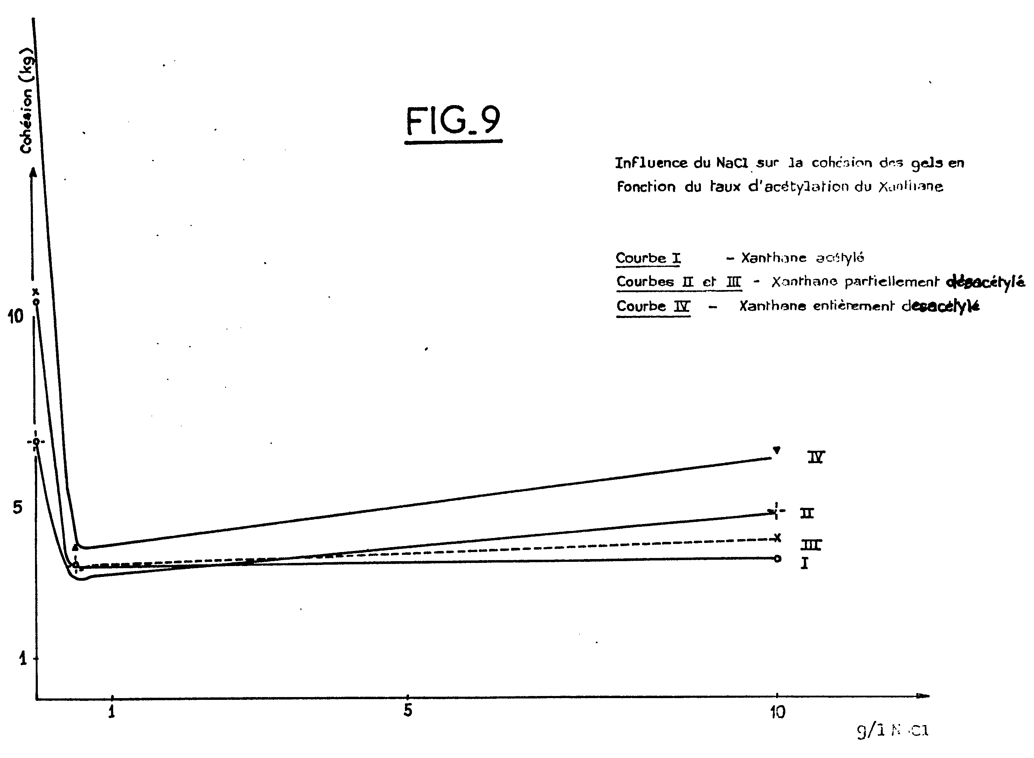

On a comparé les caractéristiques d'un xanthane acétylé et de trois échantillons de xanthane plus ou moins désacétylé obtenus à partir de ce xanthane acétylé. La figure 8 donne le résultat des mesures de rigidité et la figure 9 le résultat des mesures de cohésion.

L'avantage de la désacétylation apparaît bien aux diverses teneurs en chlorure de sodium et en fonction de l'importance de la désacétylation.

### Exemple 7

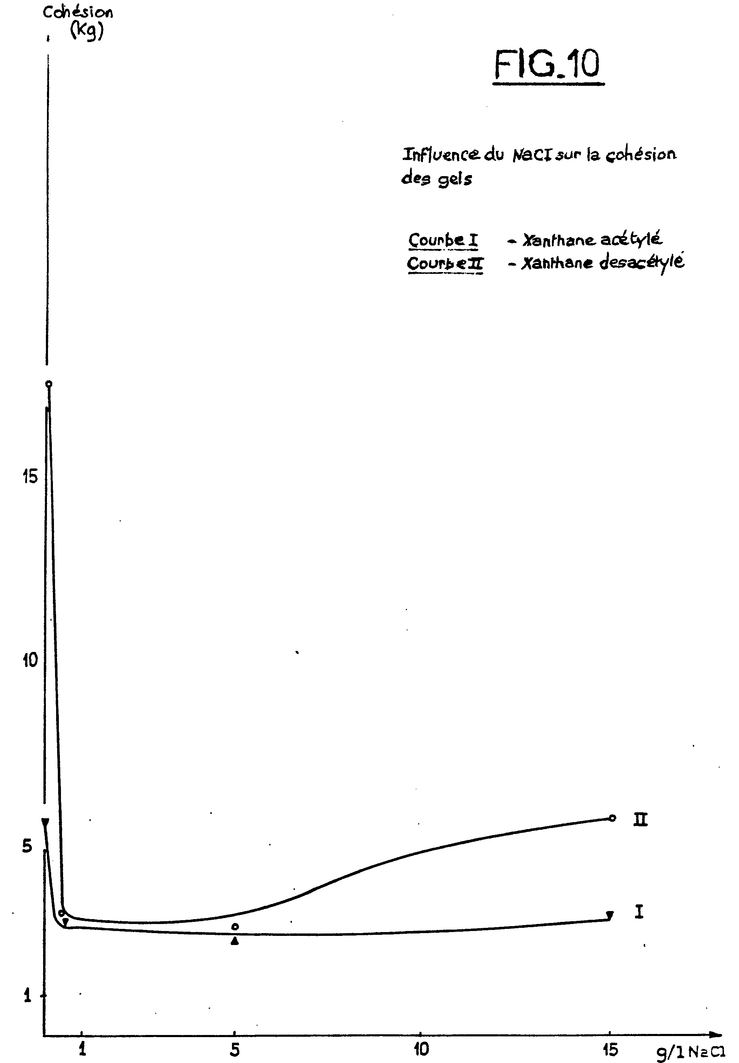

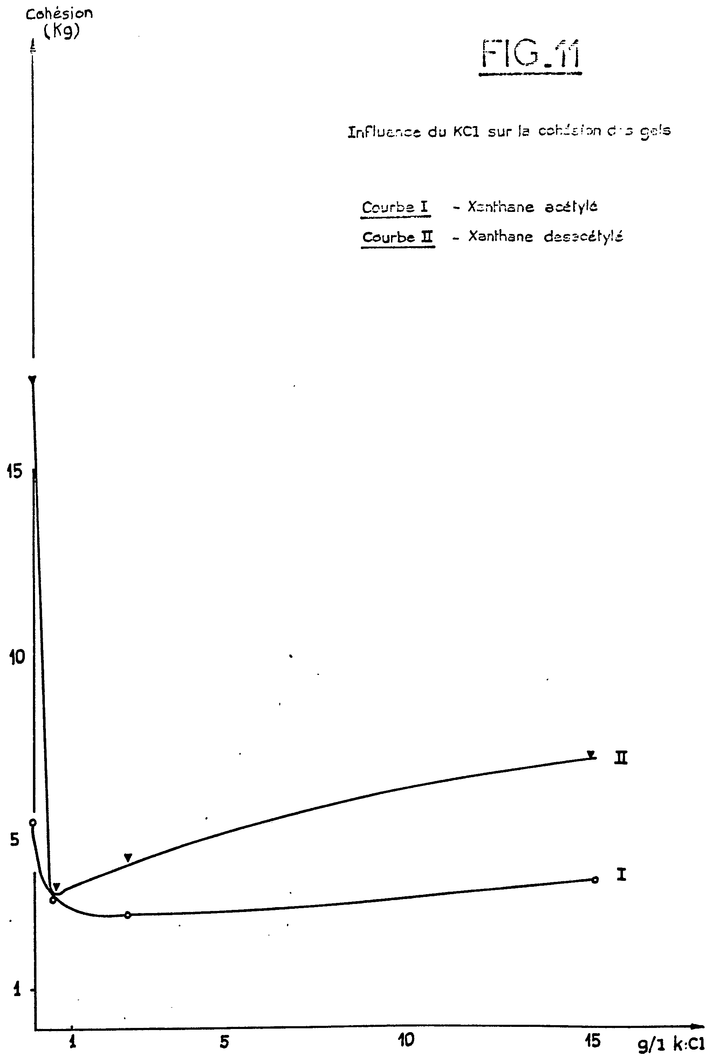

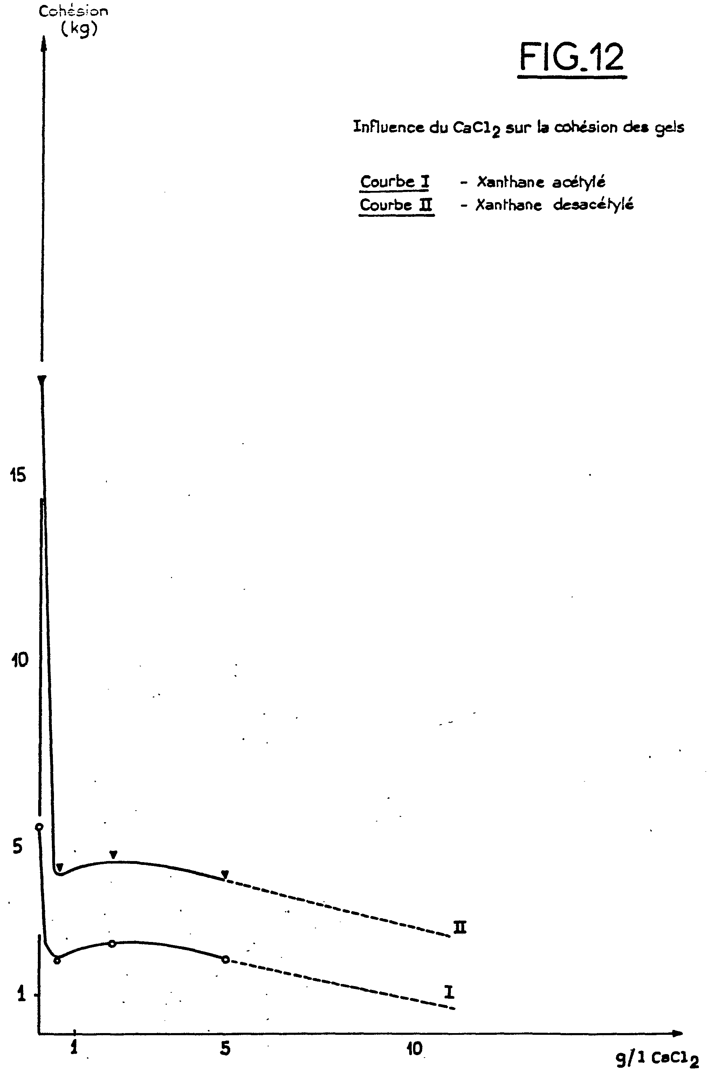

L'étude de l'influence des électrolytes a été poursuivie en comparant un échantillon de xanthane acétylé et un échantillon de xanthane désacétylé utilisés en association 50/50 avec la gomme de caroube, pour diverses teneurs en chlorure de sodium, chlorure de potassium et chlorure de calcium. Les figures 10, 11, et 12 donnent les résultats obtenus pour chacun de ces trois sels et montrent que la cohésion est plus élevée avec le xanthane désacétylé pour chacun de ces trois sels. Pour la rigidité, il en est de même ainsi que le montre le tableau VI.

### TABLEAU VI

| Sel g/l | | Xanthane acétylé | Xanthane désacétylé |
|---|---|---|---|
| Témoin 0 | | 20 | 41 |
| NaCl | 0,5 | 30 | 46 |
| | 5 | 29 | 39 |
| | 15 | 28 | 38 |
| KCl | 0,5 | 31 | 50 |
| | 5 | 35 | 48 |
| | 15 | 37 | 45 |
| CaCl$_2$ | 0,5 | 21 | 26 |
| | 2 | 21 | 27 |
| | 5 | 22 | 27 |

Exemple 8 : Influence des anions usuels sur un gel de xanthane désacétylisé et de gomme de caroube

Les anions ordinaires n'apportent pas de changement à la réactivité du xanthane désacétylé vis-à-vis de la gomme de caroube.

Le tableau VII donne les résultats obtenus avec un mélange xanthane désacétylé/gomme de caroube 50/50 auquel on ajoute divers sels de sodium en quantité telle que la proportion de Na$^+$ par rapport à la gomme soit constante et corresponde à 5 g/l de NaCl.

### TABLEAU VII

| Sel ajouté | Rigidité (g) | Cohésion (kg) |
|---|---|---|
| Témoin ........................................................ | 20 | 5,5 |
| Chlorure ..................................................... | 30 | 3 |
| Carbonate .................................................. | 25 | 2,9 |
| Nitrate ....................................................... | 32 | 3,2 |
| Acétate ...................................................... | 28 | 3,3 |

TABLEAU VII (suite)

| Sel ajouté | Rigidité (g) | Cohésion (kg) |
|---|---|---|
| Sulfate ............................................... | 33 | 3,3 |
| Monosulfate ....................................... | 29 | 2,6 |
| Citrate sodique ................................. | 30 | 2,6 |

Ce tableau montre que la réactivité du xanthane désacétylé avec les galactomannanes n'est pas affectée par les divers anions utilisés.

Les gels de xanthane désacétylé plus galactomannanes peuvent avoir une variété de consistance pratiquement infinie suivant le galactomannane employé, la proportion xanthane/galactomannane et la dose de gomme totale utilisée. Ils peuvent donc trouver application, par exemple, dans l'alimentation dans les gels de charcuterie (aspics) ou les desserts gélifiés ou dans le domaine technique dans les gels d'explosifs ou les gels pour produits de traitement d'air.

**Revendications**

1. Composition gélifiante consistant en l'association de galactomannane et de xanthane, caractérisée en ce que le xanthane est désacétylé et que le rapport pondéral xanthane désacétylé/galactomannane est compris entre 15/85 et 90/10.

2. Composition gélifiante selon la revendication 1, caractérisée en ce que le galactomannane est choisi dans le groupe constitué par les gommes de caroube, de guar, de tara, d'Espina corona, utilisées seules ou en mélange.

3. Composition gélifiante selon la revendication 2, caractérisée en ce que la gomme de caroube a un degré de polymérisation tel qu'en solution à 1 % sa viscosité mesurée à 20 °C au viscosimètre Brookfield à 20 tours/minute est comprise entre 20 et 6 000 centipoises.

4. A titre de produits nouveaux, les gels obtenus à partir des compositions selon les revendications 1 à 3, utilisés en solution dans un milieu aqueux à concentration comprise entre 0,1 et 4 %.

**Claims**

1. Gelling composition consisting of a combination of galactomannan and xanthan, characterized in that the xanthan is deacetylated and that the deacetylated xanthan/galactomannan ratio by weight lies between 15/85 and 90/10.

2. The gelling composition according to claim 1, characterized in that the galactomannan is chosen from the group formed by the carob, guar and espina corona tara gums, used alone or mixed.

3. The gelling composition according to claim 2, characterized in that the carob gum has a level of polymerization such that its viscosity in 1 % solution measured at 20 °C by the brookfield viscometer at 20 revolutions per minute lies between 20 and 6 000 centipoises.

4. As new products, the gels obtained from the compositions according to claims 1 to 3, used in solution in the aqueous phase at concentrations lying between 0.1 and 4 %.

**Ansprüche**

1. Gelbildende Zusammensetzung, bestehend aus einer Vereinigung von Galactomannan und Xanthan, dadurch gekennzeichnet, dass das Xanthan desacetyliert ist und das Gewichtsverhältnis desacetyliertes Xanthan/Galactomannan zwischen 15/85 und 90/10 liegt.

2. Gelbildende Zusammensetzung gemäss Anspruch 1, dadurch gekennzeichnet, dass das Galactomannan aus der Gruppe gewählt wird, die aus Gummiarten wie Johannisbrotkernmehl, Guar, Tara d'Espina Corona besteht, die allein oder im Gemisch eingesetzt werden.

3. Gelbildende Zusammensetzung gemäss Anspruch 2, dadurch gekennzeichnet, dass das Johannisbrotkernmehl einen solchen Polymerisationsgrad hat, dass in einer 1 %igen Lösung seine im Brookfield-Viskosimeter bei 20 °C und 20 U/Min gemessene Viskosität zwischen 20 und 6 000 Zentipoisen liegt.

4. Als neue Produkte, die aus den Zusammensetzungen gemäss den Ansprüchen 1 bis 3 erhaltenen Gele, eingesetzt in Lösung in einem wässrigen Milieu mit einer Konzentration zwischen 0,1 und 4 %.

FIG_1

# FIG.2

Cohésion (kg)

Rigidité (g)

Xanthane acétylé : **Courbe I** : Rigidité
**Courbe II** : Cohésion

Xanthane desacétylé : Courbe **III** : Rigidité
Courbe **IV** : Cohésion

Mélanges avec gomme de caroube dépolymérisée
$\eta$ 1% = 40 m Pa. s

Gels à 1% du mélange

Xanthane/Gomme de Caroube

0 001 192

# FIG_3

Courbe I   : Xanthane acétylé/gomme de guar  } Viscosité
Courbe II  : Xanthane desacétylé/Gomme de guar
Courbe III : Xanthane desacétylé/Gomme de guar : Cohésion

Viscosité ($mPa.s$)
Cohésion (kg)

Xanthane/Gomme de guar

0 001 192

FIG_4

FIG_5

Xanthane acétylé : Courbe I rigidité
($\eta$ 1% 1000 $mPa.g$) : Courbe II cohésion

Xanthane désacétylé : Courbe III rigidité
($\eta$ 1% 1000 $mPas$) : Courbe IV cohésion

Mélanges avec gomme d'Espina Corona
$\eta$ 1% = 800 $mPa.s$

Gels à 1% du mélange

0 001 192

Rigidité (g)

FIG_6

Influence du NaCl sur la rigidité des gels

Courbe I — Xanthane acétylé réf. I
Courbe II — Xanthane acétylé réf. II
Courbe III — Xanthane acétylé réf. III
Courbe IV — Xanthane désacétylé

g/1 NaCl

0 001 192

# FIG.7

Cohésion
(kg)

Influence du NaCl sur la cohésion des gels

Courbe I — Xanthane acétylé Réf. II
Courbe II — Xanthane acétylé Réf. I
Courbe III — Xanthane acétylé Réf. III
Courbe IV — Xanthane desacétylé

10

IV

I
II
III

3

1

5        10        20        30        g/1 NaCl

0 001 192

Rigidité (g)

FIG.8

Influence du NaCl sur la rigidité des gels en fonction du taux d'acétylation du Xanthane

| Courbe I | — Xanthane acétylé |
| Courbes II et III | — Xanthanes partiellement desacétylés |
| Courbe IV | — Xanthane desacétylé |

III
II
I

50

20

10

1          5          10          g/1 NaCl

0 001 192

# FIG.9

Influence du NaCl sur la cohésion des gels en
fonction du taux d'acétylation du Xanthane

Courbe I        — Xanthane acétylé
Courbes II et III — Xanthane partiellement désacétylé
Courbe IV       —   Xanthane entièrement désacétylé

Cohésion (kg)

g/1 N Cl

0 001 192

FIG.10

Influence du NaCI sur la cohésion des gels

| Courbe I | – Xanthane acétylé |
| Courbe II | – Xanthane desacétylé |

FIG. 11

Influence du KCl sur la cohésion des gels

Courbe I  – Xanthane acétylé
Courbe II  – Xanthane désacétylé

FIG.12

Influence du CaCl₂ sur la cohésion des gels

Courbe I  – Xanthane acétylé
Courbe II  – Xanthane desacétylé